# EUROPÄISCHE PATENTANMELDUNG

(11) **EP 0 854 151 A1**
(43) Veröffentlichungstag der Anmeldung: **22.07.1998**
(21) Anmeldenummer: 97100790.1
(22) Anmeldetag: 20.01.1997
(51) Int. Cl.: C07K 14/805

(54) **Verfahren zur Gewinnung einheitlicher Fraktionen hyperpolymerer Hämoglobine**

(71) Anmelder: SanguiBioTech AG, 55128 Mainz (DE)
(72) Erfinder: Barnikol, Wolfgang K.R., Prof. Dr.Dr., 55128 Mainz (DE)
(74) Vertreter: Beil, Hans Christoph, Dr.

(57) **Zusammenfassung**

Die Erfindung betrifft ein Verfahren zur Herstellung hochreiner Lösungen polymerisierter Hämoglobine, die nach der molekularen Größe fraktioniert sind, wobei zum Fällen Polyhydroxyverbindungen verwendet werden.

## Beschreibung

Die Erfindung betrifft ein Verfahren zur Gewinnung einheitlicher Fraktionen hyperpolymerer Hämoglobine.

Chemische Modifikationen nativer Hämoglobine zum Zwecke einer Veränderung der Sauerstoffaffinität oder ihres Polymerisationsgrades werden durchgeführt mit dem Ziel, einen künstlichen Sauerstoffträger zu entwickeln. Dieser Träger soll beim Menschen die Sauerstofftransportfunktion des Blutes unterstützen.

Künstliche Sauerstoffträger können beispielsweise nach einem Unfall mit schwer zu beherrschenden Blutungen oder im Falle eines Infektionsrisikos (Hepatitis, AIDS) als Ersatz für eine momentan nicht verfügbare, passende Blutkonserve infundiert werden. Dies gilt auch dann, wenn ein Mensch beispielsweise in einen Volumenmangelschock geraten ist. Möglicherweise kann ein künstlicher Sauerstoffträger einen Volumenmangelschock eher durchbrechen als eine Blutkonserve, weil die konservierten Erythrozyten mit zunehmender Lagerzeit mehr und mehr versteifen und deshalb eine verringerte Kapillardurchgängigkeit aufweisen, da sie sich während der Kapillarpassage stark verformen müssen. Solche künstlichen Sauerstoffträger weisen keine Blutgruppenantigene auf. Sie könnten daher universell und ohne vorangehende Kreuzprobe eingesetzt werden.

Gegenüber einer Blutkonserve bietet darüber hinaus ein künstlicher Sauerstoffträger für den Fall einen Vorteil, daß trotz ABO- und Rhesuskompatibilität, die Gefahr einer immunologischen Überreaktion gegenüber Leukozyten besteht: ein solcher Sauerstoffträger ist dagegen völlig frei von weißen Blutkörperchen, welche diese Reaktionen auslösen können. Filtrationen der Blutkonserven, über Baumwolle beispielsweise, erweisen sich zur Elimination der weißen Blutzellen als unzureichend. Mit Tierversuchen wurde schon vor vielen Jahren gezeigt, daß mit künstlichen Sauerstoffträgern ein Volumenmangelschock wirksamer bekämpft werden kann als mit einfachen Plasmaexpandern (Übersichtsartikel hierzu: R. Pabst, Med. Klin. 72 (1977), 1555-1562).

Weiterhin ist zu erwarten, daß auch chronische Durchblutungsstörungen koronarer, cerebraler und peripherer Art mit Hilfe geeigneter Polyhämoglobinlösungen wirksam behandelt werden können. Nicht zuletzt lassen sich Sauerstoffmangelzustände ohne Durchblutungsverminderung, wie chronische Anämien, mit solchen Lösungen effektiv behandeln. Dafür spricht, daß oxygeniertes Hämoglobin seinen Sauerstoff frei gelöst besser abgeben kann als 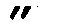 abgepackt 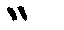 in Erythrozyten oder in Liposomen (siehe unten). Diese Indikationen bilden schätzungsweise das zehnfache Marktpotential eines "Blutersatzes" in Form der Volumensubstitution. In solchen Fällen muß der künstliche Sauerstoffträger als hyponkotisches Additiv, als "Blutzusatz", verabreicht werden.

Zur Herstellung künstlicher Sauerstoffträger sind bereits verschiedene Wege beschritten worden, nämlich
1. Die Verwendung von Emulsionen von Fluorkohlenwasserstoffen, in welchen der Sauerstoff sehr gut löslich ist (Übersicht hierzu: Issues from the V^{th} International Symposium on Blood Substitutes, Artificial Cells, Blood Substitutes, and Immobilization Biotechnology 22 (1994). Diese Methode hat jedoch den Nachteil, daß bei Anwendung von Fluorkohlenwasserstoffen gewebliche Reaktionen durch makrozytäre Zellen auftreten; entscheidend ist hier eine kritische Größe der Vesikel, die bei 0,3 µm liegt.
2. Die Mikroverkapselung konzentrierter Hämoglobinlösungen in Phospholipidvesikeln zu sogenannten künstlichen Erythrozyten oder 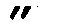 Hämosomen" (Übersichtsartikel hierzu: Gaber et al., Encapsulation of hemoglobin in Phospholipid Vesicles; Preparation and Properties of a Red Cell Surrogate in "The Red Cell. Sixth Ann Arbor Conference", G.J. Brewer (Herausgeber), Alan R. Liss, Inc. New York, (1984), 179-190). Hier besteht die Gefahr einer makrozytären Aktivierung. Wie unter 1 ist ein Wechsel der künstlichen Emulgatoren in die (dynamischen) Membranen der Zellen wahrscheinlich, so daß die Funktion dieser Membranen gestört werden kann.
3. Herstellung geeigneter Hämoglobinlösungen, auch unter kovalenter Bindung des Hämoglobins an Dextran, wodurch die renale Ausscheidung reduziert werden soll; Dextrane neigen jedoch zur Stimulation des Immunsystems.
4. Eine Vernetzung der Hämoglobinmoleküle untereinander verhindert ebenfalls die renale Ausscheidung. Zusätzlich besteht gegenüber den dextrangebundenen Hämoglobinen der Vorteil einer höheren Sauerstoffbindungskapazität. In neuerer Zeit ist es durch Vernetzung gelungen, lösliche hyperpolymere Hämoglobine zu erhalten, deren kolloidosmotischer Druck bei Vorliegen der erforderlichen Konzentrationen gegenüber dem normalen Druck (32mbar) zu vernachlässigen ist (Pötzschke et al., Advances in Experimental Medicine and Biology; N. Back et al. (Eds) Vol 345 205-213 Plenum Press New York 1994). Dieser Ansatz vereint in sich die meisten der genannten Vorteile.

Die verschiedenen genannten Ansätze spiegeln sich in nachfolgend genannten Patenten.

Die Schrift DE-OS 24 17 619 beschreibt beispielsweise die Synthese polymerisierten, verknüpften Hämoglobins als Plasmaproteinersatz, wobei mit Dicarboxilidat verknüpftes Hämoglobin hergestellt wird.

Die Schrift DE-OS 27 14 252 beschreibt die Herstellung mit Pyridoxalphosphat verknüpften Hämoglobins.

Die Schrift DE-OS 30 29 307 betrifft einen künstlichen Träger, der durch kovalente Verknüpfung eines Polysaccharids, beispielsweise Dextran, mit zellfreiem Hämoglobin hergestellt wird.

Die Schrift BE-PS 838 933 beschreibt die Herstellung eines wasserlöslichen, verknüpften, polymerisierten Hämoglobins durch Umsetzung freien Hämoglobins mit einem polyfunktionell verknüpfenden Agens und anschließendem Abstoppen der Reaktion mit inaktivierenden Agenzien. Es wird ein polymeres Hämoglobin mit einem Molekulargewicht von 64 000 bis 1 000 000 Dalton hergestellt.

Das Patent US 40 01 401 betrifft ein verknüpftes, polymerisiertes Hämoglobin als Plasmaexpander mit einem Molekulargewicht von 64 000 bis 1 000 000 Dalton, das mittels der verknüpfenden Agenzien Glutaraldehyd, Hexamethylendiisocyanat oder Butadiendiepoxid gewonnen wird.

Das Europäische Patent EP 0 201 618 beschreibt ein Verfahren zur Herstellung extrer hochmolekularer, kompakter, löslicher Polymerer des Hämoglobins aus hoch konzentrierten Lösungen monomeren Hämoglobins. Diese hochmolekularen Polymere werden als Hyperpolymere bezeichnet.

In der Schrift DE-PS 37 14 351 ist dieses Verfahren insofern vereinfacht, als Erythrozyten direkt Verwendung finden, und das Vernetzungsmittel nicht mehr in einer Lipidphase zugesetzt werden muß.

Bei der Präparation geeigneter modifizierter Hämoglobinlösungen für eine klinisch-praktische Nutzung tritt unter anderem die Notwendigkeit auf, die Viskosität der Lösung möglichst gering zu halten. Die Viskosität des Blutes bestimmt entscheidend den sogenannten totalen peripheren Widerstand des Organismus mit. Wird jener zu groß, dann toleriert das der Kreislauf nicht mehr; insbesondere tritt eine zusätzliche Belastung des Herzens auf. Im Falle gelöster Hyperpolymerer und auch Hämoglobinpolymerer, treten solche Probleme verstärkt auf, wenn eine Polymerisation zu Kettenmolekülen erfolgt.

Das Einsteinsche Viskositätsgesetz besagt, daß einheitlich große Kugeln in einer Flüssigkeit, unabhängig von ihrem Radius, eine minimale Viskosität aufweisen; diese hängt allein vom globulären Volumenanteil der Kugeln an der Lösung ab. Damit die Viskosität der Lösungen gering bleibt, müssen demgemäß die Polymermoleküle so kompakt, sprich so "kugelig" sein, daß bei geringster Viskosität des Plasmas, eine möglichst große Menge des Sauerstoffträgers transportiert werden kann.

Daher ist es zur Verringerung der Viskosität wichtig, möglichst einheitliche sauerstofftragende Moleküle herstellen zu können. Solche Moleküle finden sich auch in der Natur z.B. beim Regenwurm. Die Einheitlichkeit der Moleküle sowie das Erreichen eines möglichst hohen Molekulargewichts ist Voraussetzung für einen künstlichen Sauerstoffträger, der die Forderung nach einem vernachlässigbaren kolloidosmotischen Druck erfüllt. Um dies zu erreichen, müssen zumindest alle niedermolekularen Anteile des betreffenden hyperpolymeren Hämoglobins entfernt werden.

Mit der EP-PS 0 201 618 und der DE-PS 37 14 351 kann das Problem der Verknüpfung von Hämoglobin zu kompakten, aber löslichen, Riesenmolekülen als gelöst angesehen werden. Die dort beschriebenen Verfahren führen jedoch zu einem Hyperpolymergemisch mit einer breiten Verteilung des Molekulargewichts und einer stark überproportionalen Zunahme der Viskosität bei steigender Konzentration (Barnikol und Burkhard, Adv. Exp. Biol. Med. 248 (1989) 335 - 340), insbesondere in demjenigen Konzentrationsbereich, in dem der künstliche Träger angewendet werden soll.

Die Aufgabe der vorliegenden Erfindung ist es deshalb, ein Verfahren zu schaffen, mit dem aus einer bekannten Hämoglobinhyperpolymerlösung mit breiter Verteilung der Molekulargewichte molekular einheitliche Hämoglobinhyperpolymere aber verschiedenen Molekulargewichts abgetrennt werden können.

Dadurch ließe sich das Molekulargewicht eines künstlichen Sauerstoffträgers sehr hoch einstellen, um die Forderung nach einem vernachlässigbaren kolloidosmostischen Druck erfüllen zu können. Tatsächlich kann durch Abtrennung der Monomeren und Oligomeren mittels Ultrafiltration die Viskosität bereits gesenkt werden (Barnikol, Burkhard siehe oben); jedoch ist das Maß der Senkung unzureichend.

DE 38 41 105 C2 beschreibt ein Verfahren zur Gewinnung molekular-einheitlicher Fraktionen des polymerisierten Hämoglobins, das sich einer fraktionierten Lösung bedient. Hierbei macht man sich zunutze, daß das polymere Hämoglobin während der Vernetzungsreaktion zunächst ausfällt, aber einige Stunden später wieder praktisch vollständig in Lösung geht, und zwar überraschenderweise zunächst die niedermolekularen Polymeren. Es zeigte sich jedoch, daß die erhaltenen Lösungsfraktionen eine unzureichende Einheitlichkeit aufweisen, was der Notwendigkeit einer niedrigen Viskosität der Polyhämoglobinlösung sowie eines ausreichend geringen onkotischen Druckes entgegen steht. Idealerweise werden möglichst einheitliche Polymere aus etwa 10-20 monomeren Einheiten, d.h. Hämoglobinmolekülen, benötigt.

DE 44 18 973 A1 beschreibt ein Verfahren zur Herstellung molekular einheitlicher Hämoglobine durch die Kombination verschiedener Trennprozesse, nämlich Ultrafiltration, fraktionierte Fällung mit Ammoniumsulfat, Chromatographie und partielles Lösen. Die Ultrafiltration ist jedoch ein zusätzlicher aufwendiger technischer Prozeß, der die Denaturierung des empfindlichen Moleküls steigert - und der - im Hinblick auf eine Produktion mit großer Ausbeute - auch den Nachteil besitzt, daß er das Verfahren verteuert. Zudem wird durch die kombinierte Anwendung der genannten Verfahren die vermehrte Bildung von Dyshämoglobinen (z.B. Methämoglobin), welche Sauerstoff nicht mehr zu binden vermögen, gefördert. Weiterhin verringert sich durch viele Arbeitsschritte die Ultrafiltration die Ausbeute erheblich, so daß die Produktion sehr kostenintensiv wird, zumal das Ausgangsmaterial für die Vernetzungsreaktion hochgereinigtes Hämoglobin sein müßte.

DE 44 21 742 A1 beschreibt ein Verfahren zur Herstellung molekular einheitlicher Hämoglobine durch stufenweise Umsetzung mit einem bi- oder multifunktionellen Vernetzungsagens. Fünf Dimerisierungsschritte sind mindestens nötig, um ein ausreichend großes Molekulargewicht erhalten zu können. Die in jedem Schritt erforderlichen chromatographischen Trennungsverfahren lassen jedoch die Ausbeute nahezu exponentiell abnehmen, abgesehen von einer starken Zunahme des Anteils an Dyshämoglobin.

Die Verfahren des Standes der Technik, einheitliche hyperpolymere Hämoglobine zu gewinnen, besitzen somit die Nachteile aufwendiger technischer Prozeßführung, Denaturierung der empfindlichen Moleküle, Bildung unerwünschter Nebenprodukte, Heterogenität der Molekulargewichtsverteilung, unzureichendem Polymerisationsgrad, Verunreinigung mit Monomeren und Oligomeren und aufwendige Reinigungsverfahren. Und schließlich ist teilweise ihre Ausbeute zu gering.

Demgegenüber liegt vorliegender Erfindung die Aufgabe zugrunde, ein Verfahren zur Herstellung eines modifizierten, polymerisierten Hämoglobins zu liefern, das ein stabiles, hochvernetztes, möglichst einheitliches hyperpolymeres Hämoglobin mit hoher Sauerstofftransportfähigkeit und geringem Dyshämoglobingehalt großer Ausbeute liefert; es sollte technisch einfach sowie preiswert sein, ein hohes Trennvermögen besitzen und für alle Molekulargewichte der polymeren Hämoglobine einheitliche Fraktionen der Polymeren liefern können.

Es bestand also die Notwendigkeit, ein Fraktionierungsverfahren mit den genannten Eigenschaften zu entwickeln.

Bekannte Verfahren der Fraktionierung können aus genannten Gründen nicht zur Anwendung kommen. Die Gelchromatographie ist zudem ein teures Verfahren, welches darüber hinaus nur geringe Ausbeuten liefert. Eine fraktionierte Fällung von Proteinen mit Alkoholen, wie sie zur Gewinnung bestimmter Plasmaproteine durchgeführt wird, ist nur dann erfolgreich, wenn es sich um verschiedene Proteine einheitlichen Molekulargewichts und gleicher Molekülform handelt; das Verfahren führt nach eigenen Versuchen im Fall vernetzter Hämoglobine nicht zum Erfolg. Gleiches gilt für die bekannte Ammoniumsulfat-Fällung.

Die obige Aufgabe wird erfindungsgemäß dadurch gelöst, daß die hyperpolymeren Hämoglobine mit Polyethylenglykol fällbar sind. Insbesondere stellte sich überraschenderweise heraus, daß dieser Polyalkohol eine fraktionierte Fällung der hyperpolymeren Hämoglobine erlaubt, die zu den gewünschten engen Fraktionen dieser Polymeren führt. Hieraus ergibt sich, daß die Löslichkeit der vernetzten Hämoglobine in Polyethylenglykol/Wasser-Gemischen stark vom Verknüpfungsgrad der kovalent verkoppelten Hämoglobine abhängt. Die Erfindung wird nun an nachfolgenden Anwendungsbeispielen demonstriert.

### Anwendungsbeispiel 1: Vernetzung des Hämoglobins

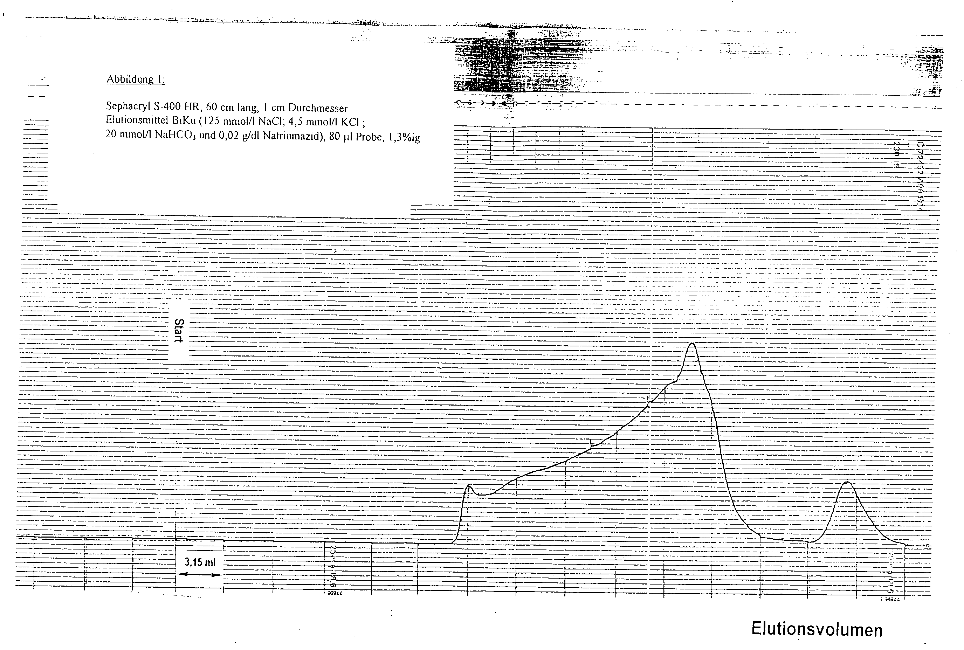

In diesem Anwendungsbeispiel handelt es sich um Rinderhämoglobin, welches mit Diisothiocyanatobenzene-Sulfat (DIBS) vernetzt wird. Heparinisiertes Rinderblut (25 000 I.E. pro 500 ml) wird dreimal mit 0,15 M NaHCO₃ gewaschen und anschließend 30 min. lang bei 20 000 g zentrifugiert. Die gepackten Erythrozyten werden durch Gefrierschock lysiert; das ergibt eine etwa 30%ige Hämoglobinlösung. 200 ml dieser Lösung lassen sich durch Rühren und Überströmen mit befeuchtetem Stickstoff in 24 h bei 4 °C desoxygenieren. Nach Zugabe einer zehnfach molaren Menge DIBS, gelöst in Dimethylsulfoxid, vernetzt sich das Hämoglobin in 16 Stunden, danach Beenden der Reaktion mit Lysin-HCl bei pH = 8,3 (2 ml 1M pro 0,45 g Hb), anschließend Lösen des Polymeren mit destilliertem Wasser 24 h lang im zehnfachen Probevolumen. Abbildung 1 zeigt das Chromatogramm des auf diese Weise hergestellten Ausgangsproduktes für die Fällungen.

### Anwendungsbeispiel 2: Abtrennen der hochmolekularen Anteile vom Ausgangsprodukt (siehe Anwendungsbeispiel 1) durch Fällung mit Polyethylenglykol 6000 (PEG)

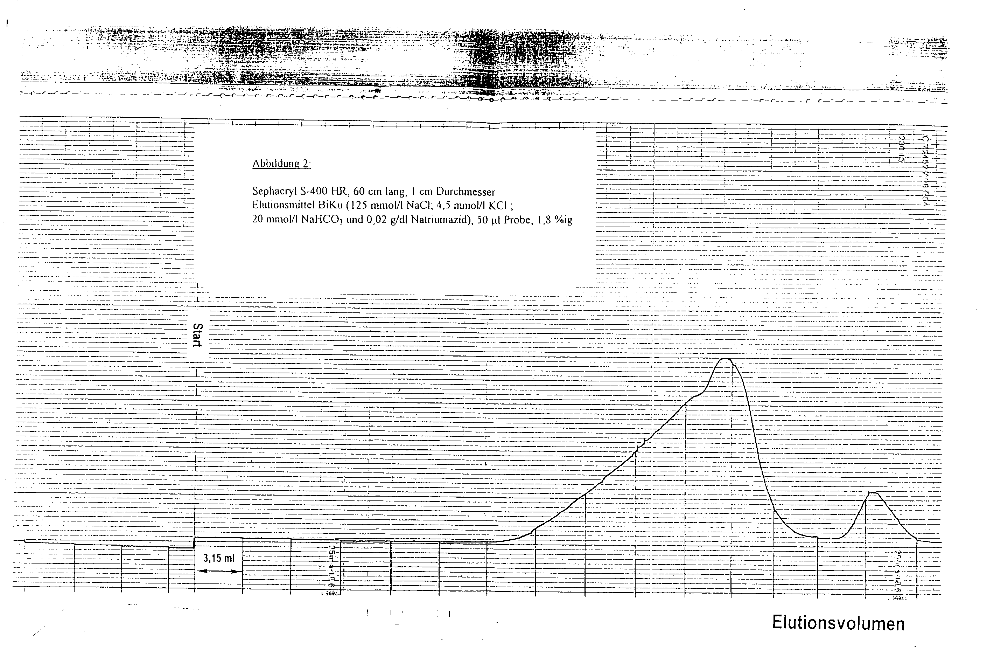

43,5 g einer 2,7%igen Hb-Lösung des Ausgangsproduktes (pH = 7,9) wurden mit 6,5 g einer Lösung PEG (50 g PEG und 50 g BiKu) versetzt, gemischt und 2 Stunden lang bei 4 °C fällen lassen. Danach erfolgte eine Zentrifugation 30 min. bei 15 000 U/min.. Die Ausbeute des Überstands betrug 70%. Abb. 2 zeigt das Chromatogramm des Hämoglobinpolymeren im Überstand.

### Anwendungsbeispiel 3: Fraktionierte Fällung mit PEG aus dem Überstand des Anwendungsbeispieles 2

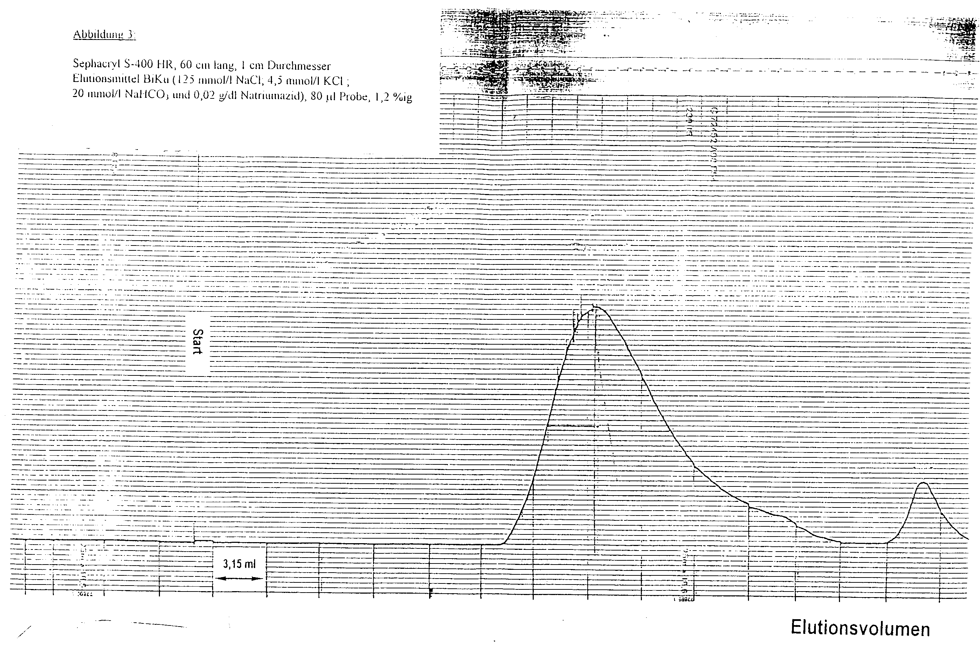

Zu 9,82 g des Überstandes wurden 0,18 ml einer PEG-Lösung (50 g PEG und 50 g BiKu) gegeben und die Mischung bei 4 °C 16 Stunden reagieren lassen. Danach erfolgte scharfes Zentrifugieren, 1maliges Waschen des Präzipitates mit etwa 12 %iger PEG-Lösung in BiKu, dann Lösen des Präzipitates unter Rühren in Biku. Abb. 3 zeigt ein Chromatogramm des gelösten Präzipitats.

### Biku : 1

25 mmol/l NaCl; 4,5 mmol/l KCl; 20 mmol/l NaHCO₃ und 0,02 g/dl Natriumazid

## Patentansprüche

1. Verfahren zur Herstellung hochreiner Lösungen polymerisierter Hämoglobine, die nach der molekularen Größe fraktioniert sind, wobei zum Fällen Polyhydroxyverbindungen verwendet werden.

2. Verfahren gemäß Anspruch 1, gekennzeichnet durch Verwenden von Polyalkoholen als Polyhydroyverbindungen.

3. Verfahren gemäß Anspruch 2, gekennzeichnet durch Verwenden von Polyethylenglykolen als Polyalkoholen.
